(19) Europäisches Patentamt
European Patent Office
Office européen des brevets

(11) Veröffentlichungsnummer: 0 226 885
A1

(12) EUROPÄISCHE PATENTANMELDUNG

(21) Anmeldenummer: 86116656.9

(22) Anmeldetag: 01.12.86

(51) Int. Cl.4: C07D 239/38 , C07D 405/12 , A01N 43/54

(30) Priorität: 13.12.85 DE 3544208

(43) Veröffentlichungstag der Anmeldung: 01.07.87 Patentblatt 87/27

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Sasse, Klaus, Dr.
Pützweg 13
D-5060 Bergisch Gladbach 2(DE)
Erfinder: Hagemann, Hermann, Dr.
Kandinsky-Strasse 52
D-5090 Leverkusen 1(DE)
Erfinder: Santel, Hans-Joachim, Dr.
Grünstrasse 9a
D-5090 Leverkusen 1(DE)
Erfinder: Schmidt, Robert R., Dr.
Im Waldwinkel 110
D-5060 Bergich Gladbach 2(DE)

(54) **Pyrimidylmercapto-acylanilide.**

(57) Die Erfindung betrifft Pyrimidylmercapto-acylanilide der Formel (I),

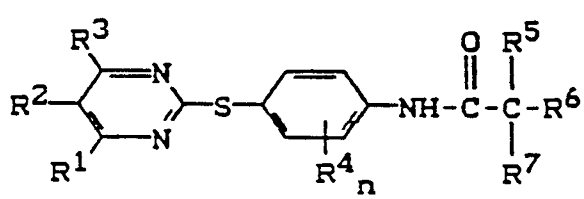

(I)

in welcher
$R^1$ für Methyl oder Ethyl steht,
$R^2$ für Halogen, Methyl oder Ethyl steht,
$R^3$ für Wasserstoff oder Methyl steht,
$R^4$ für Halogen, Methyl oder Methoxy steht,
n für 0, 1 oder 2 steht,

EP 0 226 885 A1

## Pyrimidylmercapto-acylanilide

Die vorliegende Erfindung betrifft neue Pyrimidylmercaptoacylanilide, mehrere Verfahren zu ihrer Herstellung und ihre Verwendung als Herbizide.

Es ist bereits bekannt, daß bestimmte Carbonsäureanilide herbizide Eigenschaften besitzen (vgl. R. Wegler "Chemie der Pflanzenshutz-und Schädlingsbekämpfungsmittel" Bd. 2, Seiten 311-314, Springer-Verlag, Berlin 1970). So kann zum Beispiel das Propionsäure-3,4-dichloranilid zur Unkrautbekämpfung eingesetzt werden. Die Wirkung dieser Verbindung ist gut, jedoch werden bei geringen Aufwandmengen einige Unkräuter nicht immer voll erfaßt. Außerdem läßt auch die Selektivität in manchen Fällen zu wünschen übrig.

Ferner ist bekannt, daß zahlreiche Pyrimidinyl-2-ether und thioether als Herbizide geeignet sind (vgl. JP-OS 9 474/1967, US-PS 3 126 271 und US-PS 3 250 775). Zum Beispiel können das 2-Phenoxy-4,6-dimethyl-pyrimidin und das 2-(4-Chlorbenzylmercapto)-4,6-dimethyl-pyrimidin zur Bekämpfung von Unkräutern verwendet werden. Die herbizide Potenz diese Stoffe ist aber nicht immer ausreichend.

Weiterhin ist bekannt, daß niedere Acylderivate von 4-Pyridyloxy-(bzw. thio)-anilinen herbizide Eigenschaften aufweisen (vgl. DE-OS 2 501 648, JP-OS 55 122 763 und JP-OS-56 123 970). Darüberhinaus sind auch herbizid wirksame Acylderivate von 4-Pyrimidyloxy-anilinen bekannt, die in der 5-Stellung des Pyrimidylrestes durch Halogen oder Trifluormethyl substituiert sind, dagegen in den Positionen 4 und 6 keinen Substituenten enthalten (vgl. JP-OS-56 029 576). Auch die Wirksamkeit dieser Stoffe ist jedoch für praktische Zwecke nicht immer befriedigend.

Es wurden nun neue Pyrimidylmercapto-acylanilide der Formel (I),

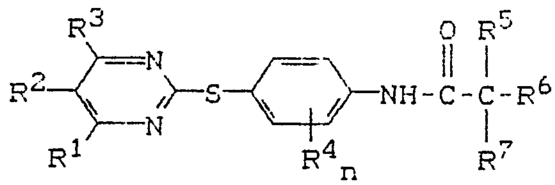

(I)

in welcher

$R^1$ für Methyl oder Ethyl steht,

$R^2$ für Halogen, Methyl oder Ethyl steht,

$R^3$ für Wasserstoff oder Methyl steht,

$R^4$ für Halogen, Methyl oder Methoxy steht,

n für 0, 1 oder 2 steht,

$R^5$ für Wasserstoff, Halogen, Cyano, gegebenenfalls substituiertes Alkyl mit 1 bis 6 Kohlenstoffatomen, gegebenenfalls substituiertes Aryl, gegebenenfalls substituiertes Aralkyl oder für die Reste $-OR^8$ oder $-SO_m-R^8$ steht, wobei

$R^8$ für gegebenenfalls substituiertes Alkyl mit 1 bis 6 Kohlenstoffatomen oder für gegebenenfalls substituiertes Aryl steht und

m für 0, 1 oder 2 steht,

und

$R^6$ und $R^7$ unabhängig voneinander für Halogen oder gegebenenfalls substituiertes Alkyl mit 1 bis 6 Kohlenstoffatomen stehen oder

$R^5$ und $R^6$ gemeinsam mit dem angrenzenden Kohlenstoffatom für einen gegebenenfalls substituierten, gesättigten oder ungesättigten Ring mit 3 bis 8 Ringgliedern stehen, der außer Kohlenstoffatomen auch Sauerstoff-und Schwefelatome enthalten kann, oder

$R^6$ und $R^7$ gemeinsam mit dem angrenzenden Kohlenstoffatom für einen gegebenenfalls substituierten gesättigten oder ungesättigten Ring mit 3 bis 8 Ring-Kohlenstoffatomen stehen,

gefunden.

Weiterhin wurde gefunden, daß man Pyrimidylmercapto-acylanilide der Formel (I) erhält, wenn man

a) Anilin-Derivate der Formel (II),

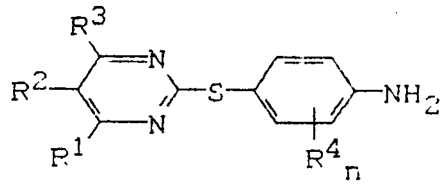

(II)

in welcher

$R^1$, $R^2$, $R^3$, $R^4$ und n die oben angegebene Bedeutung haben,

entweder

α) mit Säurehalogeniden der Formel (IIIa),

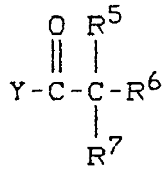

(IIIa)

in welcher

$R^5$, $R^6$ und $R^7$ die oben angegebene Bedeutung haben

und

Y für Halogen steht,

gegebenenfalls in Gegenwart eines Verdünnungsmittels und gegebenenfalls in Gegenwart eines Säurebindemittels umsetzt,

oder

β) mit symmetrischen Carbonsäureanhydriden der Formel (IIIb),

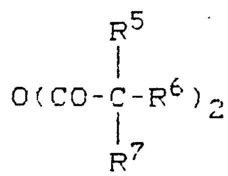

(IIIb)

in welcher

$R^5$, $R^6$ und $R^7$ die oben angegebene Bedeutung haben,

gegebenenfalls in Gegenwart eines Verdünnungsmittels umsetzt,

oder

γ) mit asymmetrischen Säureanhydriden der Formel (IIIc),

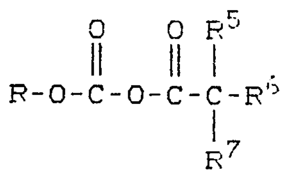

(IIIc)

in welcher

$R^5$, $R^6$ und $R^7$ die oben angegebene Bedeutung haben und

R für Alkyl mit 1 bis 4 Kohlenstoffatomen oder Phenyl steht,

gegebenenfalls in Gegenwart eines Verdünnungsmittels umsetzt,

oder

δ) mit Verbindungen der Formel (IIId),

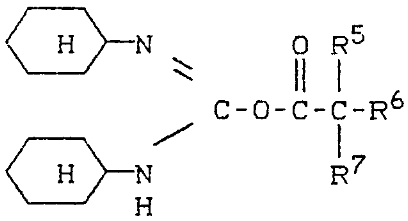

(IIId)

in welcher
$R^5$, $R^6$ und $R^7$ die oben angegebene Bedeutung haben,
gegebenenfalls in Gegenwart eines Verdünnungsmittels umsetzt,
oder wenn man
b) Pyrimidin-Derivate der Formel (IV),

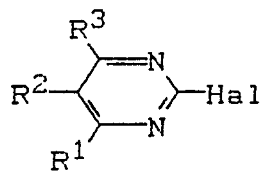

(IV)

in welcher
$R^1$, $R^2$ und $R^3$ die oben angegebene Bedeutung haben und
Hal für Halogen steht,
mit Acylanilin-Derivaten der Formel (V),

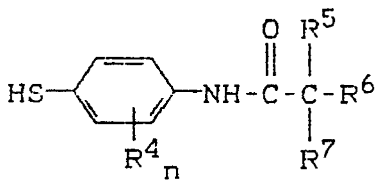

(V)

in welcher
$R^4$, $R^5$, $R^6$, $R^7$ und n die oben angegebene Bedeutung haben,
in Gegenwart eines Verdünnungsmittels umsetzt.

Schließlich wurde gefunden, daß sich die neuen Pyrimidylmetcapto-acylanilide der Formel (I) durch hervorragende herbizide Wirksamkeit auszeichnen.

Überraschenderweise besitzen die erfindungsgemäßen Pyrimidylmercapto-acylanilide der Formel (I) wesentlich bessere herbizide Eigenschaften als die konstitutionell ähnlichsten vorbekannten Stoffe. So lassen sich die erfindungsgemäßen Pyrimidylmercapto-acylanilide der Formel (I) wesentlich besser zur Unkrautbekämpfung verwenden als das 2-Phenoxy-4,6-dimethyl-pyrimidin, welches ein strukturell ähnlicher vorbekannter Wirkstoff gleicher Wirkungsart ist.

Die erfindungsgemäßen Pyrimidylmercapto-acylanilide sind durch die Formel (I) allgemein definiert. Bevorzugt sind Verbindungen der Formel (I), bei welchen
$R^1$ für Methyl oder Ethyl steht,
$R^2$ für Fluor, Chlor, Brom, Methyl oder Ethyl steht,
$R^3$ für Wasserstoff oder Methyl steht,
$R^4$ für Fluor, Chlor, Brom, Methyl oder Methoxy steht,
n für 0 oder 1 steht,
$R^5$ für Wasserstoff, Fluor, Chlor, Brom, Cyano, gegebenenfalls durch Fluor, Chlor, Brom, und/oder Alkoxy mit 1 bis 4 Kohlenstoffatomen substituiertes Alkyl mit 1 bis 4 Kohlenstoffatomen, gegebenenfalls durch Fluor, Chlor, Brom, Trifluormethyl, Methoxy und/oder Methyl substituiertes Phenyl, gegebenenfalls durch Fluor, Chlor, Brom, Trifluormethyl, Methoxy, und/oder Methyl substituiertes Benzyl oder für die Reste $-OR^8$ oder $-SO_m-R^8$ steht, wobei

$R^8$ für gegebenenfalls durch Fluor, Chlor und/oder Brom substituiertes Alkyl mit 1 bis 4 Kohlenstoffatomen oder für gegebenenfalls durch Fluor, Chlor, Brom und/oder Alkyl mit 1 bis 4 Kohlenstoffatomen substituiertes Phenyl steht und

m für 0, 1 oder 2 steht,

$R^6$ und $R^7$ unabhängig voneinander für Fluor, Chlor, Brom oder gegebenenfalls durch Fluor, Chlor und/oder Brom substituiertes Alkyl mit 1 bis 4 Kohlenstoffatomen stehen oder

$R^5$ und $R^6$ gemeinsam mit dem angrenzenden Kohlenstoffatom für einen gegebenenfalls durch Fluor, Chlor und/oder Alkyl mit 1 bis 4 Kohlenstoffatomen substituierten gesättigten oder ungesättigten Ring mit 3 bis 7 Ringatomen stehen, der außer Kohlenstoffatomen auch Sauerstoff-und Schwefelatome als Ringglieder enthalten kann oder

$R^6$ und $R^7$ gemeinsam mit dem angrenzenden Kohlenstoffatom für einen gegebenenfalls durch Fluor, Chlor und/oder Alkyl mit 1 bis 4 Kohlenstoffatomen substituierten gesättigten oder ungesättigten Ring mit 3 bis 7 Ringatomen stehen.

Eine besonders bevorzugte Gruppe erfindungsgemäßer Stoffe sind diejenigen substituierten Carbonsäureanilide der Formel (I), in denen

$R^1$ für Methyl oder Ethyl

$R^2$ für Fluor, Chlor, Methyl, oder Ethyl

$R^3$ für Wasserstoff oder Methyl

$R^4$ für Fluor, Chlor, Methyl oder Methoxy steht,

n für 0 oder 1 steht,

$R^5$ für Fluor, Chlor, Brom, Cyano, gegebenenfalls einfach bis fünffach durch Fluor, Chlor, Methoxy und/oder Ethoxy substituiertes Alkyl mit 1 bis 4 Kohlenstoff atomen, gegebenenfalls einfach bis dreifach durch Fluor, Chlor und/oder Methyl substituiertes Phenyl, gegebenenfalls einfach bis fünffach durch Fluor, Chlor und/oder Methyl substituiertes Benzyl oder für die Reste -$OR^8$ oder -$SO_m$-$R^8$ steht, worin

$R^8$ für gegebenenfalls einfach bis fünffach durch Fluor und/oder Chlor substituiertes Alkyl mit 1 bis 4 Kohlenstoffatomen oder für gegebenenfalls einfach bis fünffach durch Fluor, Chlor und/oder Alkyl mit 1 bis 4 Kohlenstoffatomen substituiertes Phenyl steht und

m für 0, 1 oder 2 steht,

$R^6$ und $R^7$ unabhängig voneinander für Fluor, Chlor oder gegebenenfalls einfach bis fünffach durch Fluor und/oder Chlor substituiertes Alkyl mit 1 bis 4 Kohlenstoffatomen stehen

oder

$R^5$ und $R^6$ gemeinsam mit dem angrenzenden Kohlenstoffatom für einen gegebenenfalls einfach bis fünffach gleich oder verschieden durch Fluor, Chlor, Methyl, Ethyl, n-Propyl, iso-Propyl, n-Butyl, iso-Butyl, sek.-Butyl und tert.-Butyl substituierten gesättigten oder ungesättigten Ring mit 3 bis 7 Ringatomen stehen, der außer Kohlenstoffatomen auch Sauerstoff-und Schwefelatome als Ringglieder enthalten kann oder

$R^6$ und $R^7$ gemeinsam mit dem angrenzenden Kohlenstoffatom für einen gegebenenfalls einfach bis fünffach gleich oder verschieden durch Fluor, Chlor, Methyl, Ethyl, n-Propyl, iso-Propyl, n-Butyl, iso-Butyl, sek.-Butyl, tert.-Butyl substituierten gesättigten oder ungesättigten Ring mit 3 bis 7 Ringatomen stehen.

Als Beispiele für substituierte Carbonsäureanilide der Formel (I) seien die in der folgenden Tabelle 1 aufgeführten Stoffe genannt.

Tabelle 1

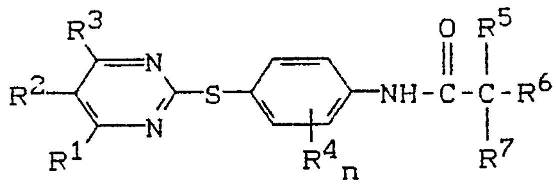

(I)

| $R^1$ | $R^2$ | $R^3$ | $R^4$ | $R^5$ | $R^6$ | $R^7$ |
|---|---|---|---|---|---|---|
| $CH_3$ | F | H | - | $CH_3$ | $CH_3$ | $CH_3$ |
| $CH_3$ | Cl | H | - | $CH_3$ | $CH_3$ | $CH_3$ |
| $CH_3$ | $CH_3$ | H | - | $CH_3$ | $CH_3$ | $CH_3$ |
| $CH_3$ | $CH_3$ | H | 2-F | $CH_3$ | $CH_3$ | $CH_3$ |
| $CH_3$ | $CH_3$ | H | 3-F | $CH_3$ | $CH_3$ | $CH_3$ |
| $CH_3$ | $CH_3$ | H | 2-Cl | $CH_3$ | $CH_3$ | $CH_3$ |
| $CH_3$ | $CH_3$ | H | 3-Cl | $CH_3$ | $CH_3$ | $CH_3$ |
| $CH_3$ | $CH_3$ | H | 2-$CH_3$ | $CH_3$ | $CH_3$ | $CH_3$ |
| $CH_3$ | $CH_3$ | H | 3-$CH_3$ | $CH_3$ | $CH_3$ | $CH_3$ |
| $CH_3$ | $CH_3$ | H | 2-$OCH_3$ | $CH_3$ | $CH_3$ | $CH_3$ |
| $CH_3$ | $CH_3$ | H | 3-$OCH_3$ | $CH_3$ | $CH_3$ | $CH_3$ |
| $CH_3$ | $CH_3$ | H | - | $CH_2F$ | $CH_3$ | $CH_3$ |
| $CH_3$ | $CH_3$ | H | - | $CH_2Cl$ | $CH_3$ | $CH_3$ |
| $CH_3$ | $CH_3$ | H | - | $C_2H_5$ | $CH_3$ | $CH_3$ |
| $CH_3$ | $CH_3$ | H | - | $C_3H_7$ | $CH_3$ | $CH_3$ |
| $CH_3$ | $CH_3$ | H | - | $OCH_3$ | $CH_3$ | $CH_3$ |
| $CH_3$ | $CH_3$ | H | - | $CH_2$-$OCH_3$ | $CH_3$ | $CH_3$ |
| $CH_3$ | $CH_3$ | H | - | 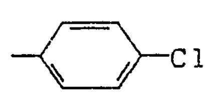 | $CH_3$ | $CH_3$ |
| $CH_3$ | $CH_3$ | H | - | 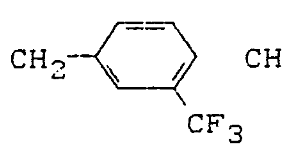 | $CH_3$ | $CH_3$ |

| $R^1$ | $R^2$ | $R^3$ | $R^4$ | $R^5$ | $R^6$ | $R^7$ |
|---|---|---|---|---|---|---|
| $CH_3$ | $CH_3$ | H | - | $CH_3$ | $-CH_2-CH_2-$ | |
| $CH_3$ | $CH_3$ | H | - | $CH_3$ | $-(CH_2)_4-$ | |
| $CH_3$ | $CH_3$ | H | - | $CH_3$ | $-(CH_2)_5-$ | |
| $CH_3$ | $C_2H_5$ | H | - | $CH_3$ | $CH_3$ | $CH_3$ |
| $C_2H_5$ | $CH_3$ | H | - | $CH_3$ | $CH_3$ | $CH_3$ |
| $C_2H_5$ | $C_2H_5$ | H | - | $CH_3$ | $CH_3$ | $CH_3$ |
| $CH_3$ | F | $CH_3$ | - | $CH_3$ | $CH_3$ | $CH_3$ |
| $CH_3$ | Cl | $CH_3$ | - | $CH_3$ | $CH_3$ | $CH_3$ |
| $CH_3$ | Br | $CH_3$ | - | $CH_3$ | $CH_3$ | $CH_3$ |
| $CH_3$ | $CH_3$ | $CH_3$ | - | $CH_3$ | $CH_3$ | $CH_3$ |
| $CH_3$ | $C_2H_5$ | $CH_3$ | - | $CH_3$ | $CH_3$ | $CH_3$ |
| $CH_3$ | Cl | $CH_3$ | 2-F | $CH_3$ | $CH_3$ | $CH_3$ |
| $CH_3$ | Cl | $CH_3$ | 3-F | $CH_3$ | $CH_3$ | $CH_3$ |
| $CH_3$ | Cl | $CH_3$ | 2-Cl | $CH_3$ | $CH_3$ | $CH_3$ |
| $CH_3$ | Cl | $CH_3$ | 3-Cl | $CH_3$ | $CH_3$ | $CH_3$ |
| $CH_3$ | Cl | $CH_3$ | 2-Br | $CH_3$ | $CH_3$ | $CH_3$ |
| $CH_3$ | Cl | $CH_3$ | $2-CH_3$ | $CH_3$ | $CH_3$ | $CH_3$ |
| $CH_3$ | Cl | $CH_3$ | $3-CH_3$ | $CH_3$ | $CH_3$ | $CH_3$ |
| $CH_3$ | Cl | $CH_3$ | $2-OCH_3$ | $CH_3$ | $CH_3$ | $CH_3$ |
| $CH_3$ | Cl | $CH_3$ | $3-OCH_3$ | $CH_3$ | $CH_3$ | $CH_3$ |
| $CH_3$ | $CH_3$ | $CH_3$ | - | $CH_2Cl$ | $CH_3$ | $CH_3$ |
| $CH_3$ | $CH_3$ | $CH_3$ | - | $CH_2F$ | $CH_3$ | $CH_3$ |
| $CH_3$ | $CH_3$ | $CH_3$ | - | H | $CH_3$ | $CH_3$ |
| $CH_3$ | $CH_3$ | $CH_3$ | - | $C_2H_5$ | $CH_3$ | $CH_3$ |
| $CH_3$ | $CH_3$ | $CH_3$ | - | $C_3H_7$ | $CH_3$ | $CH_3$ |
| $CH_3$ | $CH_3$ | $CH_3$ | - | $OCH_3$ | $CH_3$ | $CH_3$ |
| $CH_3$ | $CH_3$ | $CH_3$ | - | $CH_2-OCH_3$ | $CH_3$ | $CH_3$ |
| $CH_3$ | $CH_3$ | $CH_3$ | - | $SCH_3$ | $CH_3$ | $CH_3$ |

| $R^1$ | $R^2$ | $R^3$ | $R^4$ | $R^5$ | $R^6$ | $R^7$ |
|---|---|---|---|---|---|---|
| $CH_3$ | $CH_3$ | $CH_3$ | - | 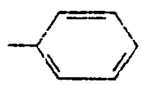 | $CH_3$ | $CH_3$ |
| $CH_3$ | $CH_3$ | $CH_3$ | - | 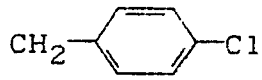 | $CH_3$ | $CH_3$ |
| $CH_3$ | $CH_3$ | $CH_3$ | - | $CH_3$ | $-CH_2-CH_2-$ | |
| $CH_3$ | $CH_3$ | $CH_3$ | - | $CH_3$ | $-(CH_2)_4-$ | |
| $CH_3$ | $CH_3$ | $CH_3$ | - | $CH_3$ | $-(CH_2)_5-$ | |

Verwendet man 4-(5-Chlor-4,6-dimethyl-pyrimidyl-2-mercapto)-anilin und Pivalsäurechlorid als Ausgangsstoffe, so kann der Verlauf des erfindungsgemäßen Verfahrens (a, Variante α durch das folgende Formelschema wiedergegeben werden:

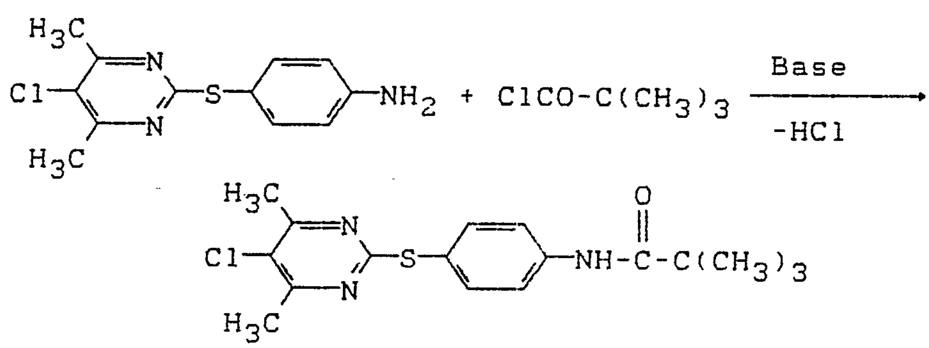


Verwendet man 4-(4,5-Dimethyl-pyrimidyl-2-mercapto)-anilin und Isobuttersäureanhydrid als Ausgangsstoffe, so kann der Verlauf des erfindungsgemäßen Verfahrens (a, Variante β) durch das folgende Formelschema wiedergegeben werden:

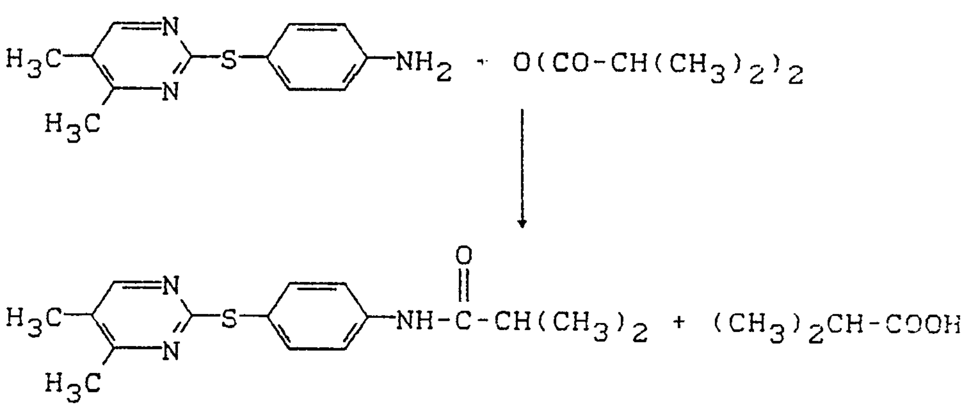

Verwendet man 4-(4,5,6-Trimethyl-pyrimidyl-2-mercapto)-anilin und α-Methoxy-isobuttersäure-kohlensäure-ethylester-anhydrid als Ausgangsstoffe, so kann der Verlauf des erfindungsgemäßen Verfahrens (a, Variante γ) durch das folgende Formelschema wiedergegeben werden:

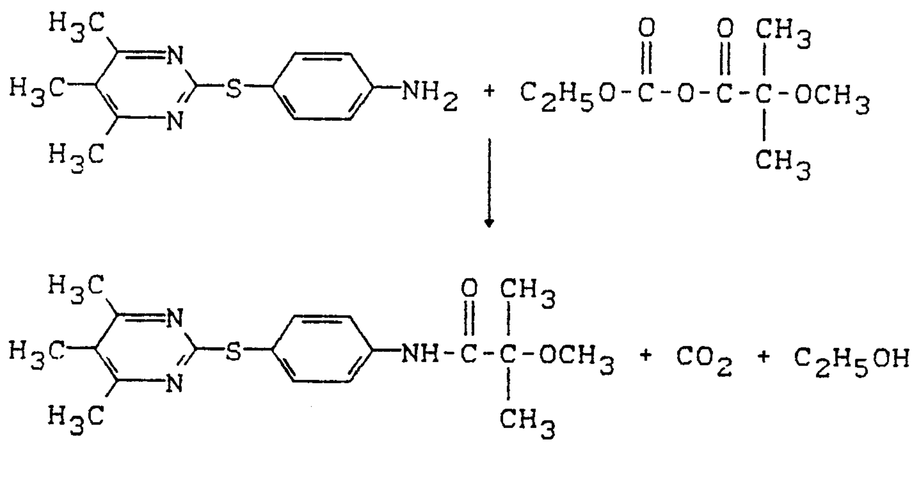

Verwendet man 4-(4-Methyl-5-fluor-pyrimidyl-2-mercapto)-anilin und O-Pivaloyloxy-dicyclohexyl-isoharnstoff als Ausgangsstoffe, so kann der Verlauf des erfindungsgemäßen Verfahrens (a, Variante δ) durch das folgende Formelschema wiedergegeben werden:

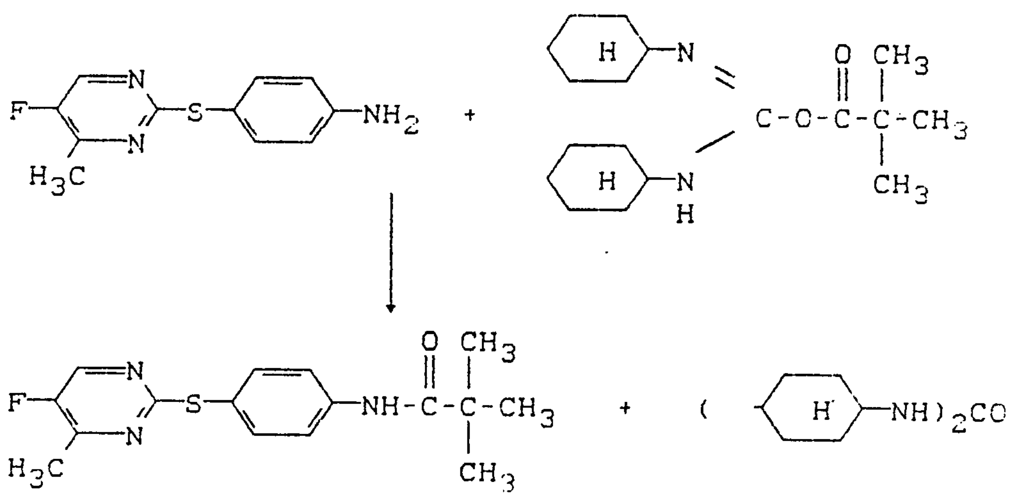

Verwendet man 2-Chlor-4-methyl-5-ethyl-pyrimidin und 4-Pivaloylamino-thiophenol als Ausgangsstoffe, so kann der Verlauf des erfindungsgemäßen Verfahrens (b) durch das folgende Formelschema wiedergegeben werden:

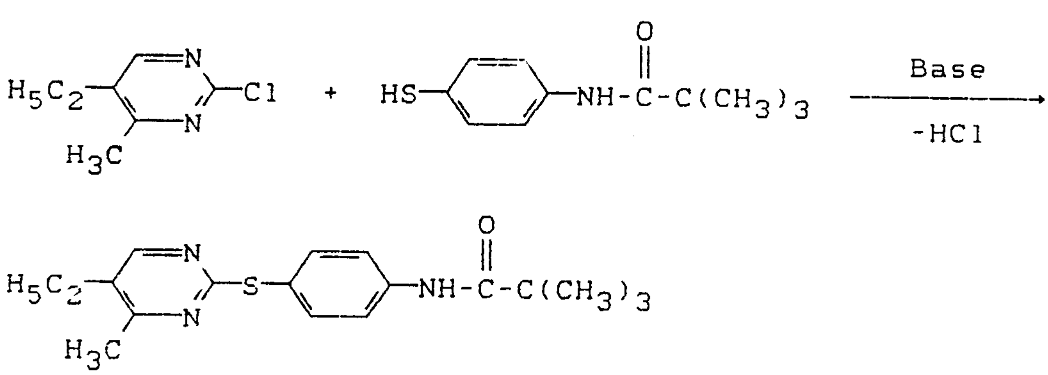

Die bei dem erfindungsgemäßen Verfahren (a) als Ausgangsstoffe benötigten Anilin-Derivate sind durch die Formel (II) allgemein definiert. In dieser Formel haben $R^1$, $R^2$, $R^3$, $R^4$ und n vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden.

Die Anilin-Derivate der Formel (II) sind teilweise bekannt. Sie lassen sich herstellen, indem man

c) Pyrimidin-Derivate der Formel (IV),

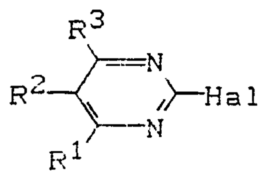

(IV)

in welcher

$R^1$, $R^2$ und $R^3$ die oben angegebene Bedeutung haben und

Hal für Halogen steht,

mit 4-Amino-thiophenolen der Formel (VI),

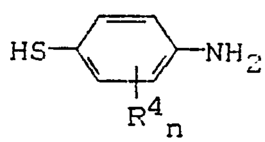

(VI)

in welcher

$R^4$ und n die oben angegebene Bedeutung haben,

in Gegenwart eines Säurebindemittels sowie gegebenenfalls in Gegenwart eines Verdünnungsmittel umsetzt,

oder

d) 2-(4-Nitro-thiophenoxy)-pyrimidin-Derivate der Formel (VII),

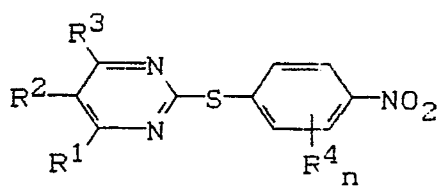

(VII)

in welcher

$R^1$, $R^2$, $R^3$, $R^4$ und n die oben angegebene Bedeutung haben,

gegebenenfalls in Gegenwart eines Verdünnungsmittels nach üblichen Methoden reduziert.

Die bei dem obigen Verfahren (c) als Ausgangsstoffe benötigten Pyrimiden-Derivate sind durch die Formel (IV) definiert. In dieser Formel haben $R^1$, $R^2$ und $R^3$ vorzugsweise diejenigen Bedeutungen, die bereits im Zusammenhang mit der Beschreibung der erfindungsgemäßen Stoffe der Formel (I) vorzugsweise für diese Reste genannt wurden. Hal steht vorzugsweise für Fluor, Chlor oder Brom.

Als Beispiele für Pyrimidin-Derivate der Formel (IV) seien genannt:

2,5-Difluor-4-methyl-pyrimidin
2,5-Dichlor-4-methyl-pyrimidin
2-Chlor-4,5-dimethyl-pyrimidin
2-Chlor-4-methyl-5-ethyl-pyrimidin
2-Chlor-4-ethyl-5-methyl-pyrimidin
2-Chlor-4,5-diethyl-pyrimidin
2,5-Difluor-4,6-dimethyl-pyrimidin

2,5-Dichlor-4,6-dimethyl-pyrimidin
2-Chlor-5-brom-4,6-dimethyl-pyrimidin
2-Chlor-4,5,6-trimethyl-pyrimidin
2-Chlor-4,6-dimethyl-5-ethyl-pyrimidin.

Die Pyrimidin-Derivate der Formel (IV) sind bekannt oder lassen sich nach im Prinzip bekannten Methoden in einfacher Weise herstellen. So erhält man Pyrimidin-Derivate der Formel (IV) zum Beispiel dadurch, daß man 2-Hydroxypyrimidin-Derivate (Dihydro-pyrimidon-2-Derivate) mit anorganischen Säurehalogeniden, wie zum Beispiel Phosphoroxychlorid oder Phosphorpentachlorid, umsetzt, oder auch dadurch, daß man entsprechende 2-Amino-pyrimidin-Derivate mit salpetriger Säure in Gegenwart von Halogenwasserstoffsäuren umsetzt.

Die bei dem Verfahren (c) weiterhin als Ausgangsstoffe benötigten 4-Amino-thiophenole sind durch die Formel (VI) definiert. In dieser Formel haben $R^4$ und n vorzugsweise diejenigen Bedeutungen, die bereits im Zusammenhang mit der Beschreibung der erfindungsgemäßen Stoffe der Formel (I) vorzugsweise für diese Reste bzw. für diesen Index genannt wurden.

Als Beispiele für 4-Amino-thiophenole der Formel (VI) seien genannt:
4-Amino-thiophenol
2-Fluor-4-amino-thiophenol
3-Fluor-4-amino-thiophenol
2-Chlor-4-amino-thiophenol
3-Chlor-4-amino-thiophenol
2-Brom-4-amino-thiophenol
3-Brom-4-amino-thiophenol
2-Methyl-4-amino-thiophenol
3-Methyl-4-amino-thiophenol
2-Methoxy-4-amino-thiophenol
3-Methoxy-4-amino-thiophenol.

Die 4-Amino-thiophenole der Formel (VI) sind bekannt oder lassen sich nach im Prinzip bekannten Methoden in einfacher Weise herstellen.

Als Säurebindemittel können bei der Durchführung des Verfahrens (c) alle üblicherweise für derartige Umsetzungen verwendbaren Säureakzeptoren verwendet werden. Vorzugsweise verwendbar sind Alkali-und Erdalkalioxide, -hydroxide und -carbonate, wie Natriumhydroxid, Kaliumhydroxid, Calciumoxid, Natriumcarbonat und Kaliumcarbonat, ferner Alkali-alkoholate, -amide und -hydride, wie zum Beispiel Natriummethylat, Natriumethylat, Kalium-tert.-butylat, Natrium-amid und Natrium-hydrid.

Als Verdünnungsmittel können bei der Durchführung des Verfahrens (c) alle üblichen inerten organischen Solventien verwendet werden. Vorzugsweise in Frage kommen Kohlenwasserstoffe, wie Benzin, Toluol und Xylol, ferner Ether, wie Dioxan, Glykoldimethylether und Diglykoldimethylether, außerdem Nitrile, wie Acetonitril, und auch stark polare Solventien, wie Dimethylsulfoxid, Sulfolan und Dimethylformamid.

Die Reaktionstemperaturen können bei der Durchführung des Verfahrens (c) innerhalb eines größeren Bereiches variiert werden. Im allgemeinen arbeitet man bei Temperaturen zwischen 0°C und 200°C, vorzugsweise zwischen 50°C und 150°C.

Die Umsetzung nach dem Verfahren (c) wird im allgemeinen unter Normaldruck vorgenommen.

Bei der Durchführung des Verfahrens (c) setzt man die Ausgangsstoffe der Formeln (IV) und (VI) im allgemeinen in angenähert äquimolaren Mengen um. Es ist jedoch auch möglich, die eine oder andere Komponente in einem größeren Überschuß zu verwenden. Die Aufarbeitung erfolgt nach üblichen Methoden.

Die bei dem Verfahren (d) als Ausgangsstoffe benötigten 2-(4-Nitro-thiophenoxy)-pyrimidin-Derivate sind durch die Formel (VII) definiert. In dieser Formel haben $R^1$, $R^2$, $R^3$, $R^4$ und n vorzugsweise diejenigen Bedeutungen, die bereits im Zusammenhang mit der Beschreibung der erfindungsgemäßen Stoffe der Formel (I) vorzugsweise für diese Reste bzw. für diesen Index genannt wurden.

Die Verbindungen der Formel (VII) sind bekannt oder lassen sich nach im Prinzip bekannten Methoden in einfacher Weise herstellen. So erhält man Verbindungen der Formel (VII) zum Beispiel dadurch, daß man Pyrimidin-Derivate der Formel (IV),

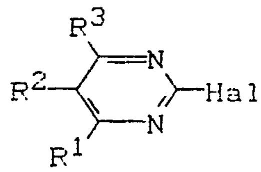

(IV)

in welcher
$R^1$, $R^2$, $R^3$ und Hal die oben angegebene Bedeutung haben,
mit 4-Nitro-thiophenolen der Formel (IX),

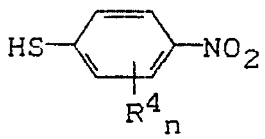

(IX)

in welcher
$R^4$ und n die oben angegebene Bedeutung haben,
in Gegenwart eines Säurebindemittels und gegebenenfalls in Gegenwart eines Verdünnungsmittels bei Temperaturen zwischen 0°C und 200°C, vorzugsweise zwischen 50°C und 150°C, umsetzt. Als Säurebindemittel und Verdünnungsmittel kommen hierbei vorzugsweise diejenigen Stoffe in Betracht, die bereits im Zusammenhang mit dem Verfahren (c) als vorzugsweise verwendbare Säureakzeptoren und Solventien genannt wurden.

Als Reduktionsmittel kommen bei dem Verfahren (d) alle diejenigen Stoffe in Frage, die üblicherweise zur Reduktion aromatischer Nitroverbindungen eingesetzt werden. Vorzugsweise verwendbar sind elementare Metalle, wie Eisen, Zink und Zinn, ferner Metallverbindungen in niederen Wertigkeitsstufen, wie Eisen (II)-und Zinn (II)-Salze, und außerdem Nichtmetall-Verbindungen in niederen Wertigkeitsstufen, wie z.B. Salze des Schwefelwasserstoffes, Alkalisulfite und Alkalidithionite. Im übrigen kann die Reduktion auch durch katalytische Hydrierung mit Wasserstoff in Gegenwart eines Katalysators, wie z.B. Raney-Nickel, erfolgen.

Als Verdünnungsmittel kommen bei dem Verfahren (d) alle üblichen für derartige Reduktionen geeigneten organischen Solventien in Betracht. Die Reaktionstemperaturen können innerhalb eines größeren Bereiches variiert werden. Sie entsprechen den Temperaturen, die bei analogen Reaktionen angewandt werden.

Die Durchführung der Reduktion nach dem Verfahren (d) und die Aufarbeitung des anfallenden Reaktionsgemisches erfolgen nach üblichen Methoden.

Die bei dem erfindungsgemäßen Verfahren (a, Variante α) als Reaktionskomponenten benötigten Säurehalogenide sind durch die Formel (IIIa) eindeutig definiert. In dieser Formel haben $R^5$, $R^6$ und $R^7$ vorzugsweise diejenigen Be deutungen, die bereits im Zusammenhang mit der Beschreibung der erfindungsgemäßen Stoffe der Formel (I) vorzugsweise für diese Reste genannt wurden. Y steht vorzugsweise für Fluor, Chlor oder Brom.

Als Beispiele für Säurehalogenide der Formel (IIIa) seien die Chloride der folgenden Säuren genannt:
Trichloressigsäure
Trifluoressigsäure
α,α-Dichlorpropionsäure
Isobuttersäure
α-Chlor-isobuttersäure
α-Brom-isobuttersäure
α-Methoxy-isobuttersäure
α-Phenoxy-isobuttersäure
α-(4-Chlor-phenoxy)-isobuttersäure
α-(2-methyl-4-chlor-phenoxy)-isobuttersäure
α-Methylmercapto-isobuttersäure
α-Methylsulfonyl-isobuttersäure
α-Methyl-buttersäure
Pivalinsäure
β-Fluor-pivalinsäure

β-Chlor-pivalinsäure
β,β'-Difluor-pivalinsäure
β,β'-Dichlor-pivalinsäure
β,β',β''-Trifluor-pivalinsäure
β,β',β''-Trichlor-pivalinsäure
α,α-Dimethyl-valeriansäure
α-Methyl-α-ethyl-buttersäure
α,α-Dimethyl-phenylessigsäure
α,α-Dimethyl-(4-chlor-phenyl)-essigsäure
α,α-Dimethyl-(3,4-dichlor-phenyl)-essigsäure
α,α-Dimethyl-(3-trifluormethyl-phenyl)-essigsäure
α-Benzyl-isobuttersäure
α-(4-Chlor-benzyl)-isobuttersäure
α-(4-Methoxy-benzyl)-isobuttersäure
Cyclopropan-carbonsäure
1-Methyl-cyclopropan-carbonsäure
2,2-Dichlor-1-methyl-cyclopropan-carbonsäure
Cyclopentan-carbonsäure
1-Methyl-cyclopentan-carbonsäure
Cyclohexan-carbonsäure
1-Methyl-cyclohexan-carbonsäure
1-Methyl-4-isopropyl-cyclohexan-carbonsäure.

Die Säurehalogenide der Formel (IIIa) sind bekannt oder lassen sich nach im Prinzip bekannten Methoden in einfacher Weise herstellen.

Als Verdünnungsmittel können bei dem erfindungsgemäßen Verfahren (a, Variante α) alle gegenüber Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methyl-isopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (a, Variante α) alle üblichen Säureakzeptoren in Betracht. Vorzugsweise verwendbar sind tertiäre Amine, wie Triethylamin, Pyridin und N,N,-Dimethyl-anilin, ferner Erdalkalimetalloxide, wie Magnesium-und Calciumoxid, außerdem Alkali-und Erdalkali-metallcarbonate, wie Natriumcarbonat, Kaliumcarbonat und Calciumcarbonat. Es ist auch möglich, die jeweiligen Anilin-Derivate der Formel (II) gleichzeitig als Säurebindemittel zu verwenden. Dazu muß die betreffende Anilin-Verbindung dann zumindest in solcher Menge eingesetzt werden, daß freiwerdende Halogenwasserstoff gebunden werden kann.

Die Reaktionstemperaturen können bei der Durchführung des erfindungsgemäßen Verfahrens (a, Variante α) innerhalb eines größeren Bereiches variiert werden. Arbeitet man ohne Lösungsmittel und Säurebindemittel, so geht man im allgemeinen so vor, daß man die Komponenten zunächst bei Temperaturen zwischen -20°C und +20°C reagieren läßt und danach auf Temperaturen zwischen 70 und 200°C erhitzt. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (a, Variante α) wird im allgemeinen unter Normaldruck durchgeführt.

Bei der Durchführung des erfindungsgemäßen Verfahrens (a, Variante α) werden die Ausgangsstoffe der Formeln (II) und (IIIa) im allgemeinen in angenähert äquivalenten Mengen verwendet. Es ist jedoch auch möglich, die eine oder andere Komponente in einem größeren Überschuß einzusetzen.

Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt. Arbeitet man in Gegenwart von Wasser oder von mit Wasser mischbaren Solventien, so kann man auch so verfahren, daß man das Reaktionsgemisch mit Wasser verdünnt, das entstehende Gemisch absaugt oder mit einem mit Wasser wenig mischbaren organischen Lösungsmittel extrahiert, die organische Phase wäscht, einengt und den verbleibenden Rückstand gegebenenfalls üblichen Reinigungsverfahren unterwirft.

Die bei dem erfindungsgemäßen Verfahren (a, Variante $\beta$) als Reaktionskomponenten benötigten symmetrischen Carbonsäure-anhydride sind durch die Formel (IIIb) eindeutig definiert. In dieser Formel haben $R^5$, $R^6$ und $R^7$ vorzugsweise diejenigen Bedeutungen, die bereits im Zusammenhang mit der Beschreibung der erfindungsgemäßen Stoffe der Formel (I) vorzugsweise für diese Reste genannt würden.

Die symmetrischen Carbonsäureanhydride der Formel (IIIb) sind bekannt oder lassen sich nach im Prinzip bekannten Methoden in einfacher Weise herstellen.

Als Verdünnungsmittel können bei der Durchführung des erfindungsgemäßen Verfahrens (a, Variante $\beta$) vorzugsweise diejenigen Verdünnungsmittel verwendet werden, die auch bei dem Verfahren (a, Variante $\alpha$) vorzugsweise in Betracht kommen. Im übrigen kann auch ein im Überschuß eingesetztes Carbonsäureanhydrid der Formel (IIIb) gleichzeitig als Verdünnungsmittel fungieren.

Die Reaktionstemperaturen können auch bei dem erfindungsgemäßen Verfahren (a, Variante $\beta$) innerhalb eines größeren Bereiches variiert werden. Im allgemeinen arbeitet man bei Temperaturen zwischen -20°C und +150°C, vorzugsweise zwischen 0 und 100°C.

Das erfindungsgemäße Verfahren (a, Variante $\beta$) wird im allgemeinen unter Normaldruck durchgeführt.

Bei der Durchführung des erfindungsgemäßen Verfahrens (a, Variante $\beta$) werden die Ausgangsstoffe der Formeln (II) und (IIIb) im allgemeinen in angenähert äquivalenten Mengen verwendet. Es ist jedoch auch möglich, das Carbonsäureanhydrid in einem größeren Überschuß einzusetzen.

Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Die bei dem erfindungsgemäßen Verfahren (a, Variante $\gamma$) als Reaktionskomponenten benötigten asymmetrischen Säureanhydride sind durch die Formel (IIIc) eindeutig definiert. In dieser Formel haben $R^5$, $R^6$ und $R^7$vorzugsweise diejenigen Bedeutungen, die bereits im Zusammenhang mit der Beschreibung der erfindungsgemäßen Stoffe der Formel (I) vorzugsweise für diese Reste genannt wurden R steht vorzugsweise für Alkyl mit 1 oder 2 Kohlenstoffatomen oder für Phenyl.

Die asymmetrischen Säureanhydride der Formel (IIIc) sind bekannt oder lassen sich nach im Prinzip bekannten Methoden in einfacher Weise herstellen. So erhält man Verbindungen der Formel (IIIc) dadurch, daß man Carbonsäuren der Formel (III)

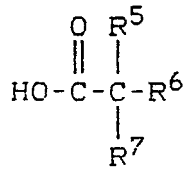 (III)

in welcher

$R^5$, $R^6$ und $R^7$ die oben angegebene Bedeutung haben,

mit Kohlensäure-ester-chloriden der Formel (X),

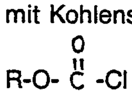 (X)

in welcher

R die oben angegebene Bedeutung hat,

in Gegenwart eines Verdünnungsmittels, wie z.B. Methylenchlorid, und in Gegenwart eines Säurebindemittels, wie z.B. Triethylamin, bei Temperaturen zwischen -20°C und +100°C, vorzugsweise zwischen 0 und 50°C, umsetzt. Die asymmetrischen Säureanhydride der Formel (IIIc) werden im allgemeinen nicht in reiner Form isoliert, sondern in der anfallenden Form, gegebenenfalls nach vorheriger Entfernung von Verdünnungsmittel und/oder Salzen, weiter verwendet.

Als Verdünnungsmittel können bei der Durchführung des erfindungsgemäßen Verfahrens (a, Variante $\gamma$) vorzugsweise diejenigen Verdünnungsmittel verwendet werden, die auch bei dem Verfahren (a, Variante $\alpha$) vorzugsweise in Betracht kommen. Im übrigen kann auch im Überschuß eingesetztes Säureanhydrid der Formel (IIIc) gleichzeitig als Verdünnungsmittel fungieren.

Die Reaktionstemperaturen können auch bei dem erfindungsgemäßen Verfahren (a, Variante $\gamma$) innerhalb eines größeren Bereiches variiert werden. Im allgemeinen arbeitet man bei Temperaturen zwischen -20°C und +150°C, vorzugsweise zwischen 0 und 100°C.

Das erfindungsgemäße Verfahren (a, Variante $\gamma$) wird im allgemeinen unter Normaldruck durchgeführt.

Bei der Durchführung des erfindungsgemäßen Verfahrens (a, Variante γ) werden die Ausgangsstoffe der Formeln (II) und (IIIc) im allgemeinen in angenähert äquivalenten Mengen verwendet. Es ist jedoch auch möglich, das Säureanhydrid in einem größeren Bereich einzusetzen. Die Aufarbeitung erfolgt nach üblichen Methoden.

Die bei dem erfindungsgemäßen Verfahren (a, Variante δ) als Reaktionskomponenten benötigten Verbindungen sind durch die Formel (IIId) eindeutig definiert. In dieser Formel haben $R^5$, $R^6$ und $R^7$ vorzugsweise diejenigen Bedeutungen, die bereits im Zusammenhang mit der Beschreibung der erfindungsgemäßen Stoffe der Formel (I) vorzugsweise für diese Reste genannt wurden.

Die Verbindungen der Formel (IIId) sind bekannt oder lassen sich nach im Prinzip bekannten Verfahren in einfacher Weise herstellen. So erhält man Verbindungen der Formel (IIId) dadurch, daß man Carbonsäuren der Formel (III),

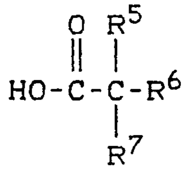

(III)

in welcher

$R^5$, $R^6$ und $R^7$ die oben angegebene Bedeutung haben,

mit Dicyclohexylcarbodiimid der Formel (XI),

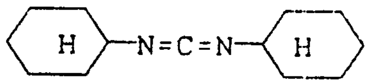

(XI)

nach üblichen Methoden umsetzt.

Als Verdünnungsmittel können bei der Durchführung des erfindungsgemäßen Verfahrens (a, Variante δ) vorzugsweise diejenigen Verdünnungsmittel verwendet werden, die auch bei dem Verfahren (a, Variante α) vorzugsweise in Betracht kommen.

Die Reaktionstemperaturen können auch bei dem erfindungsgemäßen Verfahren (a, Variante δ) innerhalb eines größeren Bereiches variiert werden. Im allgemeinen arbeitet man bei Temperaturen zwischen -20°C und +150°C, vorzugsweise zwischen 0 und 100°C.

Das erfindungsgemäße Verfahren (a, Variante δ) wird im allgemeinen unter Normaldruck durchgeführt.

Bei der Durchführung des erfindungsgemäßen Verfahrens (a, Variante δ) werden die Ausgangsstoffe der Formeln (II) und (IIId) im allgemeinen in angenähert äquivalenten Mengen verwendet. Die Aufarbeitung erfolgt nach üblichen Methoden.

Die bei dem erfindungsgemäßen Verfahren (b) als Ausgangsstoffe benötigten Pyrimiden-Derivate der Formel (IV) wurden bereits im Zusammenhang mit der Beschreibung des Verfahrens (c) abgehandelt.

Die bei dem erfindungsgemäßen Verfahren (b) weiterhin als Ausgangsstoffe benötigten Acylanilin-Derivate sind durch die Formel (V) eindeutig definiert. In dieser Formel haben $R^4$, $R^5$, $R^6$, $R^7$ und n vorzugsweise diejenigen Bedeutungen, die bereits im Zusammenhang mit der Beschreibung der Stoffe der Formel (I) vorzugsweise für diese Reste bzw. für diesen Index genannt wurden.

Die Verbindungen der Formel (V) sind bekannt oder lassen sich nach im Prinzip bekannten Methoden in einfacher Weise herstellen. So erhält man Acylanilin-Derivate der Formel (V) z.B. dadurch, daß man 4-Amino-thiophenole der Formel (VI),

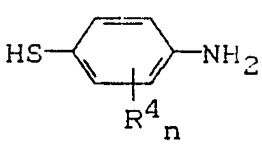

(VI)

in welcher

$R^4$ und n die oben angegebene Bedeutung haben,
mit Säurehalogeniden der Formel (IIIa)

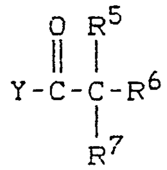

(IIIa)

in welcher
$R^5$, $R^6$, $R^7$ und Y die oben angegebene Bedeutung haben,
gegebenenfalls in Gegenwart eines Verdünnungsmittels und gegebenenfalls in Gegenwart eines Säurebindemittels umsetzt. Die Reaktionsbedingungen entsprechen dabei denjenigen, die auch bei der Durchführung des Verfahrens (a, Variante $\alpha$) angewendet werden.

Als Säurebindemittel können bei der Durchführung des erfindungsgemäßen Verfahrens (b) alle üblichen Säureakzeptoren eingesetzt werden. Vorzugsweise verwendbar sind Alkalimetall-und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, ferner Alkalimetall-und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat.

Als Verdünnungsmittel können bei dem erfindungsgemäßen Verfahren (b) alle üblichen inerten organischen Solventien eingesetzt werden. Vorzugsweise verwendbar sind Kohlenwasserstoffe, wie Toluol and Xylol, ferner Ether, wie Dibutylether, Tetrahydrofuran, Dioxan, Glykoldimethylether und Diglykoldimethylether, außerdem Nitrile, wie Acetonitril und Propionitril, und weiterhin polare Lösungsmittel, wie Nitrobenzol, Dimethylsulfoxid, Sulfolan, Dimethylformamid und N-Methyl-pyrrolidon.

Die Reaktionstemperaturen können bei der Durchführung des erfindungsgemäßen Verfahrens (b) innerhalb eines größeren Bereiches variiert werden. Im allgemeinen arbeitet man bei Temperaturen zwischen 0 und 200°C, vorzugsweise zwischen 50 und 150°C.

Das erfindungsgemäße Verfahren (b) wird im allgemeinen unter Normaldruck durchgeführt.

Bei der Durchführung des erfindungsgemäßen Verfahrens (b) setzt man die Reaktionskomponenten der Formeln (IV) und (V) im allgemeinen in etwa äquimolaren Mengen ein.

Es ist jedoch auch möglich, die eine oder andere Komponente in einem größeren Überschuß zu verwenden. Außerdem setzt man im allgemeinen auch eine äquimolare Menge an säurebindendem Mittel ein. Es kann jedoch auch von Vorteil sein, das Säurebindemittel in einem bis zu einmolaren Überschuß hinzuzufügen. Im einzelnen geht man im allgemeinen so vor, daß man des säurebindende Mittel zu einer Mischung der Reaktionskomponenten in einem geeigneten Verdünnungsmittel gibt. Man kann aber auch in der Weise verfahren, daß man zunächst aus dem Acylanilin-Derivat der Formel (V) und dem Säurebindemittel ein Salz erzeugt und dieses dann mit einem Pyrimidin-Derivat der Formel (IV) umsetzt. Weiterhin ist es auch möglich, aus dem Acylanilin-Derivat der Formel (V) mit einem Säurebindemittel zunächst separat ein Salz herzustellen, dieses dann zu isolieren und anschließend in Gegenwart eines geeigneten Verdünnungsmittels ohne weitere Zugabe eines Säurebindemittels mit einem Pyrimidin-Derivat der Formel (IV) umzusetzen. Die Aufarbeitung erfolgt jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cir sium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die erfindungsgemäßen Wirkstoffe eignen sich besonders gut zur selektiven Bekämpfung mono-und dikotyler Unkräuter in monokotylen Kulturen, wie z.B. Mais und Getreide.

Die erfindungsgemäßen Wirkstoffe weisen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Die erfindungsgemäben Wirkstoffe lassen sich mit besonderem Vorteil zur Bekämpfung von Pyricularia oryzae bei Reis, gegen Botrytis und zur Bekämpfung von Bohnenrost verwenden.

Im übrigen zeichnen sich die erfindungsgemäßen Stoffe auch durch eine insektizide Wirksamkeit aus. Sie besitzen dabei eine gute wurzelsystemische Wirksamkeit.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkysulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganischen Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 and 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethyl-harnstoff, 3-(3-Chlor-4-methylphenyl)-1,1-dimethylharnstoff, 3-(4-Isopropylphenyl)-1,1-dimethylharnstoff, 4-Amino-6-(1,1-dimethylethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H,3H)-dion, 4-Amino-3-methyl-6-phenyl-1,2,4-triazin-5-(4H)-on, 4-Amino-6-t-Butyl-3-ethylthio1,2,4-triazin-5-(4H)-on, 2-Chlor-4-ethylamino-6-isopropylamino-1,3,5-triazin, 2-Chlor-4-ethylamino-6-(3-cyanpropylamino)-1,3,5-triazin, das R-Enantiomere des 2-[4-(3,5-Dichlor-pyridin-2-oxy)-phenoxy]-propionsäure(trimethylsilyl)-methylesters, das R-Enantiomere des 2-[4-(3,5-Dichlorpyridyl-2-oxy)-phenoxy]-propionsäure-(2-benzyloxy)-ethylesters, 2,4-Dichlorphenoxyessigsäure, 2-[4-(2,4-Dichlorphenoxy)-phenoxy]-propionsäure-methylester, 4-Chlor-2-methyl-phenoxy-essigsäure, 2-(2-Methyl-4-chlorphenoxy)-propionsäure, 3,5-Dijod-4-hydroxy-benzonitril, 3,5-Dibrom-4-hydroxy-benzonitril sowie Diphenylether und Phenylpyridazine, wie z.B. Pyridate in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können bei einer Verwendung als Herbizide sowohl vor als auch nach dem Auflaufen der Pflanzen appliziert werden. Sie können auch vor der Saat in den Boden eingearbeitet werden.

Die angewandte Wirkstoffmenge kann beim Einsatz der erfindungsgemäßen Stoffe als Herbizide in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Auch beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,000 1 Gew.-%, vorzugsweise zwischen 0,5 und 0,001 Gew.-%. Bei der Saatgutbehandlung werden im allgemeinen Wirkstoffmengen von 0,001 bis 50 g je kg Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Wirkstoffe geht aus den nachfolgenden Beispielen hervor.

Herstellungsbeispiele

Beispiel 1

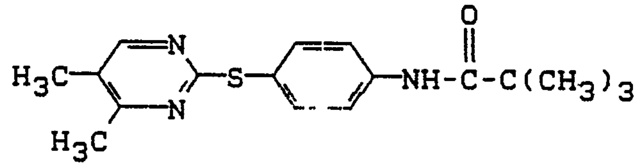

23,1 g (0,1 Mol) 2-(4-Amino-phenyl-mercapto)-4,5-dimethylpyrimidin werden in 150 ml Tetrahydrofuran gelöst. Hierzu gibt man 10,1 g (0,1 Mol) Triethylamin und tropft anschließend bei 15-20°C 12,05 g (0,1 Mol) Pivalsäurechlorid zu. Die Mischung wird 2 Stunden bei Raumtemperatur nachgerührt und dann in 1 l Wasser eingerührt. Die abgeschiedenen Kristalle werden abgesaugt, mit Wasser gewaschen und an der Luft getrocknet.

Man erhält 29,0 g (92 % der Theorie) 2-(4-Pivaloylaminophenylmercapto)-4,5-dimethylpyrimidin vom Schmelzpunkt Fp.: 156-158°C (Umkristallisation aus Waschbenzin).

Nach den im vorausgehenden Beispiel und in der Beschreibung angegebenen Methoden erhält man die in der folgenden Tabelle 2 formelmäßig aufgeführten Verbindungen der Formel (I):

Tabelle 2

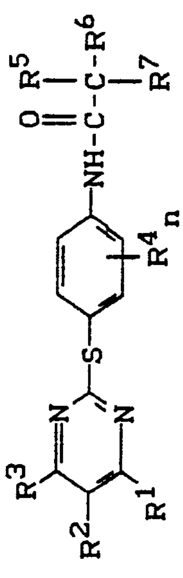

(I)

| Beispiel | $R^1$ | $R^2$ | $R^3$ | $R^4$ | $R^5$ | $R^6$ | $R^7$ | Fp. (°C) |
|---|---|---|---|---|---|---|---|---|
| 2 | $CH_3$ | $CH_3$ | H | - | $CH_2Cl$ | $CH_3$ | $CH_3$ | 122-124 |
| 3 | $CH_3$ | $CH_3$ | H | - | $C_3H_7$ | $CH_3$ | $CH_3$ | 114-116 |
| 4 | $CH_3$ | $CH_3$ | H | - | $CH_3$ | $-(CH_2)_5-$ | | 122-124 |
| 5 | $C_2H_5$ | $CH_3$ | H | - | $CH_3$ | $CH_3$ | $CH_3$ | 146-147 |
| 6 | $CH_3$ | Cl | $CH_3$ | - | $CH_3$ | $CH_3$ | $CH_3$ | 190-192 |
| 7 | $CH_3$ | $CH_3$ | $CH_3$ | - | $CH_3$ | $CH_3$ | $CH_3$ | 216-218 |
| 8 | $CH_3$ | $CH_3$ | $CH_3$ | - | $CH_2Cl$ | $CH_3$ | $CH_3$ | 200-202 |
| 9 | $CH_3$ | $C_2H_5$ | $CH_3$ | - | $CH_3$ | $CH_3$ | $CH_3$ | 188-190 |
| 10 | $CH_3$ | $C_2H_5$ | $CH_3$ | - | $CH_2Cl$ | $CH_3$ | $CH_3$ | 140-141 |
| 11 | $CH_3$ | $C_2H_5$ | $CH_3$ | - | $CH_3$ | $-(CH_2)_5-$ | | 150-152 |

Tabelle 2 (Fortsetzung)

| Beispiel | $R^1$ | $R^2$ | $R^3$ | $R^4$ | $R^5$ | $R^6$ | $R^7$ | Fp.(°C) |
|---|---|---|---|---|---|---|---|---|
| 12 | $CH_3$ | $CH_3$ | H | - | $C_2H_5$ | $C_2H_5$ | H | 171-173 |
| 13 | $CH_3$ | $CH_3$ | H | - | CN | $CH_3$ | $CH_3$ | 156-158 |
| 14 | $CH_3$ | $CH_3$ | H | - | $C_2H_5$ | $CH_3$ | $CH_3$ | 121-123 |
| 15 | $CH_3$ | $CH_3$ | H | - | $CH(CH_3)_2$ | $CH_3$ | $CH_3$ | 106-108 |
| 16 | $CH_3$ | $CH_3$ | H | - | $CH_3$ | $-CH_2-O-CH_2-$ | | 147-149 |
| 17 | $CH_3$ | $CH_3$ | H | - | $CH_3$ | $-O-(CH_2)_4-$ | | 106-108 |
| 18 | $CH_3$ | $CH_3$ | H | 3-Cl | $CH_3$ | $CH_3$ | $CH_3$ | 172-174 |
| 19 | $CH_3$ | $CH_3$ | H | 3-Cl | $CH_2Cl$ | $CH_3$ | $CH_3$ | 161-163 |
| 20 | $CH_3$ | $CH_3$ | H | 3-Cl | $C_2H_5$ | $CH_3$ | $CH_3$ | 124-125 |
| 21 | $CH_3$ | $CH_3$ | H | 3-Cl | $CH_3$ | $-(CH_2)_5-$ | | 142-144 |
| 22 | $CH_3$ | $CH_3$ | H | $3-OCH_3$ | $CH_3$ | $CH_3$ | $CH_3$ | 150-152 |
| 23 | $CH_3$ | $CH_3$ | H | $3-OCH_3$ | $CH(CH_3)_2$ | $CH_3$ | $CH_3$ | 129-131 |

Tabelle 2 (Fortsetzung)

| Beispiel | $R^1$ | $R^2$ | $R^3$ | $R^4$ | $R^5$ | $R^6$ | $R^7$ | Fp. (°C) |
|---|---|---|---|---|---|---|---|---|
| 24 | $CH_3$ | $C_2H_5$ | H | - | $CH_3$ | $CH_3$ | $CH_3$ | 128-130 |
| 25 | $CH_3$ | $C_2H_5$ | H | - | $C_2H_5$ | $CH_3$ | $CH_3$ | 124-126 |
| 26 | $CH_3$ | Cl | $CH_3$ | - | $CH_2Cl$ | $CH_3$ | $CH_3$ | 185-187 |
| 27 | $CH_3$ | Cl | $CH_3$ | - | $C_2H_5$ | $CH_3$ | $CH_3$ | 180-182 |
| 28 | $CH_3$ | Cl | $CH_3$ | - | $C_3H_7$ | $CH_3$ | | 148-150 |
| 29 | $CH_3$ | Cl | $CH_3$ | - | $CH_3$ | $-(CH_2)_5-$ | | 150-151 |
| 30 | $CH_3$ | Cl | $CH_3$ | 3-Cl | $CH_3$ | $CH_3$ | $CH_3$ | 208-209 |

Herstellung von Ausgangsverbindungen

Beispiel VII-1

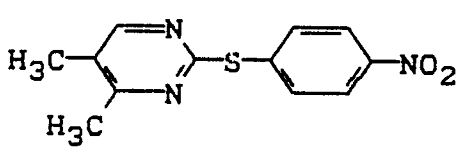

88,25 g (0,5 Mol) 4,5-Dimethyl-2-mercapto-pyrimidin-hydrochlorid werden in 1 l Sulfolan suspendiert. Bei Raumtemperatur werden unter Rühren portionsweise 70 g (1,25 Mol) pulverisiertes Kaliumhydroxid eingetragen. Man rührt 30 Minuten nach und gibt dann 79 g (0,5 Mol) 4-Chlor-nitro-benzol zu. Die Mischung wird 1 Stunde bei Raumtemperatur verrührt, anschließend 3 Stunden auf 120-130°C erwämt. Nach dem Abkühlen gießt man die Reaktionsmischung in 3 l Eiswasser ein.

Die abgeschiedenen Kristalle werden abgesaugt und an der Luft getrocknet.

Man erhält 112 g (85,8 % der Theorie) 2-(4-Nitro-phenylmercapto)-4,5-dimethyl-pyrimidin vom Schmelzpunkt Fp.: 92-94°C (Umkristallisation aus Methanol).

In entsprechender Weise gewinnt man:

Beispiel VII-2

2-(4-Nitro-phenyl-mercapto)-4-ethyl-5-methyl-pyrimidin:

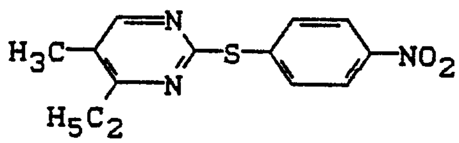

Schmelzpunkt: 70-72°C (Umkristallisation aus Ethanol).

Beispiel VII-3

2-(4-Nitro-phenyl-mercapto)-4,5,6-trimethyl-pyrimidin:

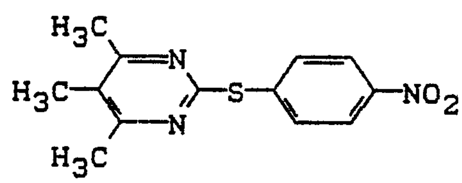

Beispiel VII-4

2-(4-Nitro-phenyl-mercapto)-5-ethyl-4,6-dimethyl-pyrimidin:

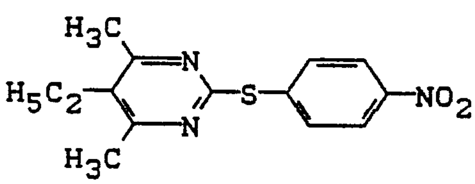

Schmelzpunkt: Fp.: 82-84°C (Umkristallisation aus Toluol).

Beispiel VII-5

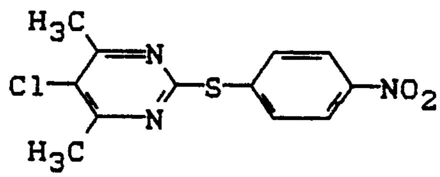

26,1 g (0,1 Mol) 2-(4-Nitro-phenyl-mercapto)-4,6-dimethyl-pyrimidin werden in 200 ml Tetrachlormethan suspendiert. Hierzu tropft man bei Raumtemperatur 14,9 g (1,1 Mol) Sulfurylchlorid. Das Gemisch wird 3 Stunden unter Rückfluß erhitzt, heiß filtriert, die Lösung im Vakuum eingedampft, der Rückstand aus Waschbenzin umkristallisiert.

Man erhält 15 g (50, 8 % der Theorie) 2-(4-Nitrophenyl-mercapto)-5-chlor-4,6-dimethyl-pyrimidin vom Schmelzpunkt Fp.: 110°C.

Beispiel II-1

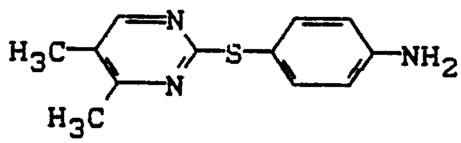

Verfahren (d)

78,3 g (0,3 Mol) 2-(4-Nitro-phenyl-mercapto)-4,5-dimethyl-pryrimidin werden in 400 ml Dioxan unter Zusatz von 15 g Raney-Nickel bei 20-50°C erschöpfend hydriert. Der Katalysator wird abgesaugt, die Lösung im Vakuum eingedampft.

Es hinterbleibt in praktisch qunatitativer Ausbeute 2-(4-Amino-phenyl-mercapto)-4,5-dimethyl-pyrimiden vom Schmelzpunkt Fp.: 126-128°C (Umkristallisation aus Toluol).

In entsprechender Weise gewinnt man:

Beispiel II-2

2-(4-Amino-phenyl-mercapto)-4-ethyl-5-methyl-pyrimidin:

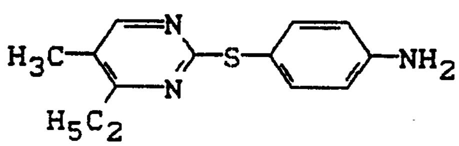

Schmelzpunkt: Fp.: 88-90°C (Umkristallisation aus Waschbenzin).

Beispiel II-3

2-(4-Amino-phenyl-mercapto)-4,5,6,-trimethyl-pyrimidin:

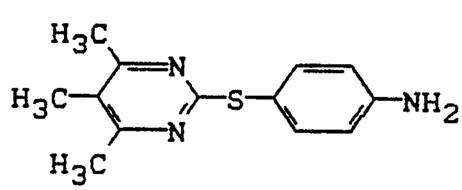

Schmelzpunkt: Fp.: 152-154°C (Umkristallisation aus Toluol).

Beispiel II-4

2-(4-Amino-phenyl-mercapto)-5-ethyl-4,6-dimethyl-pyrimidin

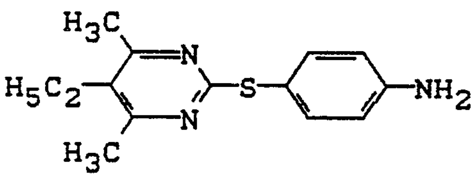

Schmelzpunkt: Fp.: 157-159°C (Umkristallisation aus Toluol).

Beispiel II-5

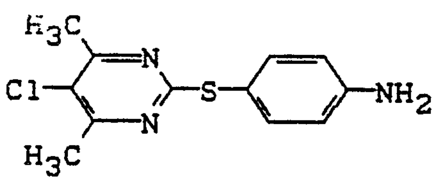

Verfahren (c)

25 g (0,2 Mol) 4-Amino-thiophenol werden in 150 ml N-Methyl-pyrrolidon gelöst. Hierzu gibt man 12,3 g (0,22 Mol) pulverisiertes Kaliumhydroxid und anschließend 35,4 g (0,2 Mol) 2,5-Dichlor-4,6-dimethyl-pyrimidin. Diese Mischung wird 5 Stunden auf 120°C erwärmt und nach dem Abkühlen in 1 Liter Wasser eingerührt. Die Kristalle werden abgesaugt, mit Wasser gewaschen und getrocknet.

Man erhält 46,2 g (87 % der Theorie) 2-(4-Amino-phenyl-mercapto)-5-chlor-4,6-dimethyl-pyrimidin vom Schmelzpunkt: 160-161°C.

Beispiel A

Post-emergence-Test

Lösungsmittel: 5 Gewichtsteile Aceton

Emulgator: 1 Gewichsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 -15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

In diesem Test zeigen die Verbindungen gemäß den Beispielen 1, 3, 4, 6, 10, 13, 14, 15, 18, 19, 20 und 25 bei der selektiven Bekämpfung von mono-und dikotylen Unkräutern, wie beispielsweise Setaria, Amaranthus und Chenopodium in monokotylen Kulturen, wie Weizen und Mais, eine sehr gute herbizide Wirksamkeit.

**Ansprüche**

1. Pyrimidylmercapto-acylanilide der Formel (I),

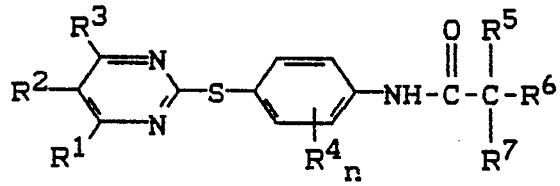

(I)

in welcher
$R^1$ für Methyl oder Ethyl steht,
$R^2$ für Halogen, Methyl oder Ethyl steht,
$R^3$ für Wasserstoff oder Methyl steht,
$R^4$ für Halogen, Methyl oder Methoxy steht,
n für 0, 1 oder 2 steht, $R^5$ für Wasserstoff, Halogen, Cyano, gegebenenfalls substituiertes Alkyl mit 1 bis 6 Kohlenstoffatomen, gegebenenfalls substiutiertes Aryl, gegebenenfalls substituiertes Aralkyl oder für die Reste $-OR^8$ oder $-SO_m-R^8$ steht, wobei
$R^8$ für gegebenenfalls substituiertes Alkyl mit 1 bis 6 Kohlenstoffatomen oder für gegebenenfalls substituiertes Aryl steht und
m für 0, 1 oder 2 steht,
und
$R^6$ und $R^7$ unabhängig voneinander für Halogen oder gegebenefalls substituiertes Alkyl mit 1 bis 6 Kohlenstoffatomen stehen oder
$R^5$ und $R^6$ gemeinsam mit dem angrenzenden Kohlenstoffatom für einen gegebenenfalls substituierten, gesättigten oder ungesättigten Ring mit 3 bis 8 Ringgliedern stehen, der außer Kohlenstoffatomen auch Sauerstoff-und Schwefelatome enthalten kann, oder
$R^6$ und $R^7$ gemeinsam mit dem angrenzenden Kohlenstoffatom für einen gegebenenfalls substituierten gesättigten oder ungesättigten Ring mit 3 bis 8 Ring-Kohlenstoffatomen stehen.

2. Pyrimidlymercapto-acylanilide der Formel (I) gemäß Anspruch 1,
in welcher
$R^1$ für Methyl oder Ethyl steht,
$R^2$ für Fluor, Chlor, Brom, Methyl oder Ethyl steht,
$R^3$ für Wasserstoff oder Methyl steht,
$R^4$ für Fluor, Chlor, Brom, Methyl oder Methoxy steht,
n für 0 oder 1 steht.
$R^5$ für Wasserstoff, Fluor, Chlor, Brom, Cyano, gegebenenfalls durch Fluor, Chlor, Brom und/oder Alkoxy mit 1 bis 4 Kohlenstoffatomen substituiertes Alkyl mit 1 bis 4 Kohlenstoffatomen, gegebenenfalls durch Fluor, Chlor, Brom, Trifluormethyl, Methoxy und/oder Methyl substituiertes Phenyl, gegebenenfalls durch Fluor, Chlor, Brom, Trifluormethyl, Methoxy und/oder Methyl substituiertes Benzyl oder für die Reste $-OR^8$ oder $-SO_m-R^8$ steht, wobei
$R^8$ für gegebenenfalls durch Fluor, Chlor und/oder Brom substituiertes Alkyl mit 1 bis 4 Kohlenstoffatomen oder für gegebenenfalls durch Fluor, Chlor, Brom und/oder Alkyl mit 1 bis 4 Kohlenstoffatomen substituiertes Phenyl steht und
m für 0, 1 oder 2 steht,
$R^6$ und $R^7$ unabhängig voneinander für Fluor, Chlor, Brom oder gegebenenfalls durch Fluor, Chlor und/oder Brom substituiertes Alkyl mit 1 bis 4 Kohlenstoffatomen stehen oder
$R^5$ und $R^6$ gemeinsam mit dem angrenzenden Kohlenstoffatom für einen gegebenenfalls durch Fluor, Chlor und/oder Alkyl mit 1 bis 4 Kohlenstoffatomen substituierten gesättigten oder ungesättigten Ring mit 3 bis 7 Ringatomen, der außer Kohlenstoffatomen auch Sauerstoff-und Schwefelatome als Ringglieder enthalten kann oder
$R^6$ und $R^7$ gemeinsam mit dem angrenzenden Kohlenstoffatom für einen gegebenenfalls durch Fluor, Chlor und/oder Alkyl mit 1 bis 4 Kohlenstoffatomen substituierten gesättigten oder ungesättigten Ring mit 3 bis 7 Ringatomen stehen.

3. Pyrimidylmercapto-acylanilide der Formel (I) gemäß Anspruch 1, in welcher
$R^1$ für Methyl oder Ethyl
$R^2$ für Fluor, Chlor, Methyl oder Ethyl
$R^3$ für Wasserstoff oder Methyl
$R^4$ für Fluor, Chlor, Methyl oder Methoxy steht,
n für 0 oder 1 steht,
$R^5$ für Fluor, Chlor, Brom, Cyano, gegebenenfalls einfach bis fünffach durch Fluor, Chlor, Methoxy und/oder Ethoxy substituiertes Alkyl mit 1 bis 4 Kohlenstoffatomen, gegebenenfalls einfach bis dreifach durch Fluor, Chlor und/oder Methyl substituiertes Phenyl, gegebenenfalls einfach bis fünffach durch Fluor, Chlor und/oder Methyl substituiertes Benzyl oder für die Reste $-OR^8$ oder $-SO_m R^8$ steht, worin
$R^8$ für gegebenenfalls einfach bis fünffach durch Fluor und/oder Chlor substituiertes Alkyl mit 1 bis 4 Kohlenstoffatomen oder für gegebenenfalls einfach bis fünffach durch Fluor, Chlor und/oder Alkyl mit 1 bis 4 Kohlenstoffatomen substituiertes Phenyl steht und
m für 0, 1 oder 2 steht,
$R^6$ und $R^7$ unabhängig voneinander für Fluor, Chlor oder gegebenenfalls einfach bis fünffach durch Fluor und/oder Chlor substituiertes Alkyl mit 1 bis 4 Kohlenstoffatomen stehen
oder
$R^5$ und $R^6$ gemeinsam mit dem angrenzenden Kohlenstoffatom für einen gegebenenfalls einfach bis fünffach gleich oder verschieden durch Fluor, Chlor, Methyl, Ethyl, n-Propyl, iso-Propyl, n-Butyl, iso-Butyl, sek.-Butyl und tert.-Butyl substituierten gesättigten oder ungesättigten Ring mit 3 bis 7 Ringatomen stehen, der außer Kohlenstoffatomen auch Sauerstoff-und Schwefelatome als Ringglieder enthalten kann oder
$R^6$ und $R^7$ gemeinsam mit dem angrenzenden Kohlenstoffatom für einen gegebenenfalls einfach bis fünffach gleich oder verschieden durch Fluor, Chlor, Methyl, Ethyl, n-Propyl, iso-Propyl, n-Butyl, iso-Butyl, sek.-Butyl, tert.-Butyl substituierten gesättigten oder ungesättigten Ring mit 3 bis 7 Ringatomen stehen.

4. Verfahren zur Herstellung von Pyrimidylmercaptoacylaniliden der Formel (I),

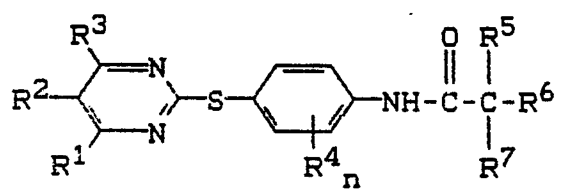

(I)

in welcher
$R^1$ für Methyl oder Ethyl steht,
$R^2$ für Halogen, Methyl oder Ethyl steht,
$R^3$ für Wasserstoff oder Methyl steht,
$R_4$ für Halogen, Methyl oder Methoxy steht,
n für 0, 1 oder 2 steht,
$R^5$ für Wasserstoff, Halogen, Cyano, gegebenenfalls substituiertes Alkyl mit 1 bis 6 Kohlenstoffatomen, gegebenenfalls substituiertes Aryl, gegebenenfalls substituiertes Aralkyl oder für die Reste $-OR^8$ oder $-SO_m-R^8$ steht, wobei
$R^8$ für gegebenenfalls substituiertes Alkyl mit 1 bis 6 Kohlenstoffatomen oder für gegebenenfalls substituiertes Aryl steht und
m für 0, 1 oder 2 steht,
und
$R^6$ und $R^7$ unabhängig voneinander für Halogen oder benenfalls substituiertes Alkyl mit 1 bis 6 Kohlenstoffatomen stehen oder
$R^5$ und $R^6$ gemeinsam mit dem angrenzenden Kohlenstoffatom für einen gegebenenfalls substituierten, gesättigten oder ungesättigten Ring mit 3 bis 8 Ringgliedern stehen, der außer Kohlenstoffatomen auch Sauerstoff-und Schwefelatome enthalten kann, oder
$R^6$ und $R^7$ gemeinsam mit dem angrenzenden Kohlenstoffatom für einen gegebenenfalls substituierten gesättigten oder ungesättigten Ring mit 3 bis 8 Ring-Kohlenstoffatomen stehen,
dadurch gekennzeichnet, daß man
a) Anilin-Derivate der Formel (II),

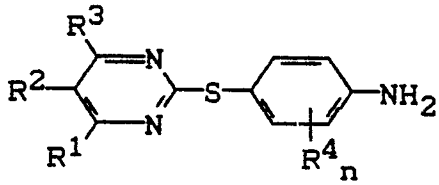

(II)

in welcher
$R^1$, $R^2$, $R^3$ $R^4$ und n die oben angegebene Bedeutung haben,
entweder
α) mit Säurehalogeniden der Formel (IIIa),

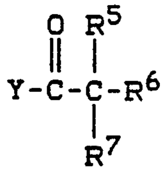

(IIIa)

in welcher
$R^5$, $R^6$ und $R^7$ die oben angegebene Bedeutung haben
und
Y für Halogen steht,
gegebenenfalls in Gegenwart eines Verdünnungsmittels und gegebenenfalls in Gegenwart eines Säurebindemittels umsetzt,
oder
β) mit symmetrischen Carbonsäureanhydriden der Formel (IIIb),

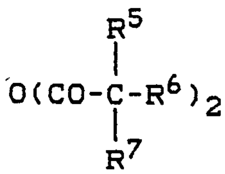

(IIIb)

in welcher
$R^5$, $R^6$ und $R^7$ die oben angegebene Bedeutung haben,
gegebenenfalls in Gegenwart eines Verdünnungsmittels umsetzt,
oder
γ) mit asymmetrischen Säureanhydriden der Formel (IIIc),

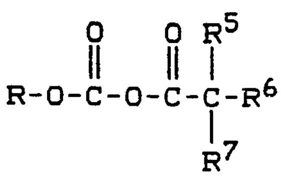

(IIIc)

in welcher
$R^5$, $R^6$ und $R^7$ die oben angegebene Bedeutung haben
und
R für Alkyl mit 1 bis 4 Kohlenstoffatomen oder Phenyl steht,
gegebenenfalls in Gegenwart eines Verdünnungsmittels umsetzt,

oder

δ) mit Verbindungen der Formel (IIId),

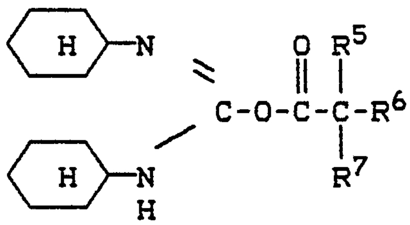

(IIId).

in welcher

$R^5$, $R^6$ und $R^7$ die oben angegebene Bedeutung haben, gegebenenfalls in Gegenwart eines Verdünnungsmittels umsetzt,

oder daß man

b) Pyrimidin-Derivate der Formel (IV),

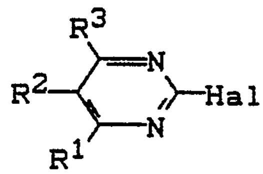

(IV)

in welcher

$R^1$, $R^2$ und $R^3$ die oben angegebene Bedeutung haben und

Hal für Halogen steht,

mit Acylanilin-Derivaten der Formel (V),

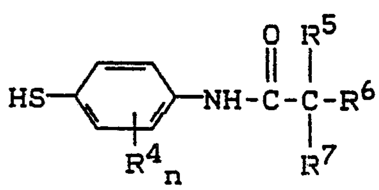

(V)

in welcher

$R^4$, $R^5$, $R^6$, $R^7$ und n die oben angegebene Bedeutung haben,

in Gegenwart eines Verdünnungsmittel umsetzt.

5. Herbizide Mittel, gekennzeichnet durch einen Gehalt an mindestens einem Pyrimidylmercapto-acylanilid der Formel (I) gemäß den Ansprüchen 1 bis 4.

6. Verfahren zur Bekämpfung von Unkräutern, dadurch gekennzeichnet, daß man Pyrimidylmercapto-acylanilide der Formel (I) gemäß den Ansprüchen 1 bis 4 auf die Unkräuter und/oder ihren Lebensraum einwirken läßt.

7. Verwendung von Pyrimidylmercapto-acylaniliden der Formel (I) gemäß den Ansprüchen 1 bis 4 zur Bekämpfung von Unkräutern.

8. Verfahren zur Herstellung von herbiziden Mitteln, dadurch gekennzeichnet, daß man Pyrimidylmercapto-acylanilide der Formel (I) gemäß den Ansprüchen 1 bis 4 mit Streckmitteln und/oder oberflächenaktiven Substanzen vermischt.

Europäisches Patentamt

# EUROPÄISCHER RECHERCHENBERICHT

Nummer der Anmeldung

EP 86116656.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,P | EP - A2 - 0 168 608 (BAYER)<br>* Ansprüche 1,3-7 *<br>-- | 1,4-8 | C 07 D 239/38<br>C 07 D 405/12<br>A 01 N 43/54 |
| A | DD - A - 109 170 (LANG et al.)<br>* Ansprüche 1-3; Tabelle 1, laufende Nr. 55 *<br>---- | 1,5-8 | |

| RECHERCHIERTE SACHGEBIETE (Int Cl.4) |
|---|
| C 07 D 239/00<br>C 07 D 405/00<br>C 07 D 409/00 |

Der vorliegende Recherchenbericht wurde für alle Patentansprüche erstellt

| Recherchenort | Abschlußdatum der Recherche | Prüfer |
|---|---|---|
| WIEN | 20-03-1987 | LUX |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82